# EUROPÄISCHE PATENTANMELDUNG

(11) **EP 4 809 951 A1**
(43) Veröffentlichungstag der Anmeldung: **23.09.2026**
(21) Anmeldenummer: 25164477.9
(22) Anmeldetag: 18.03.2025
(51) Int. Cl.: A61B 18/04, A61B 18/00

(54) **PLASMASONDE**

(71) Anmelder: Erbe Elektromedizin GmbH, 72072 Tübingen (DE)
(72) Erfinder: Schnitzler, Uwe, 72074 Tübingen (DE); Sadler, Daniel, 72074 Tübingen (DE)
(74) Vertreter: Rüger Abel IP Legal Solutions GmbH

(57) **Zusammenfassung**

Die erfindungsgemäße Plasmasonde (10) dient zur Koagulation und/oder Ablation von biologischem Gewebe eines Patienten. Sie weist einen Schlauch (15), der sich von einem proximalen Schlauchende (18) zu einem distalen Schlauchende (19) erstreckt und einen an dem distalen Schlauchende (19) angeordneten Sondenkopf (16) auf. Der Sondenkopf 16 enthält einen Elektrodenkäfig (20), der aus mehreren Elektroden (21) gebildet ist und sich von einem proximalen Kopfende (22) zu einem distalen Kopfende (23) erstreckt. Die Elektroden (21) nehmen eine Vorzugsposition ein, in der sie zumindest in einem Abschnitt (42) seitlich über den Außenumfang (25) des Schlauches (15) hinausragen. Der Sondenkopf (16) weist außerdem ein zentrales Mittelelement (27) und ein Zentrierelement (28) auf, wobei das Mittelelement (27) eine Mittelachse (24) des Sondenkopfes (16) festlegt. An dem distalen Kopfende (23) ist das Zentrierelement (28) angeordnet. Die Elektroden (21) des Elektrodenkäfigs (20) sind entlang der Mittelachse (24) relativ zu dem Mittelelement (27) verschiebbar.

## Beschreibung

Die Erfindung betrifft eine Plasmasonde zur (großflächigen) Koagulation oder Ablation von biologischem Gewebe eines menschlichen oder tierischen Patienten.

Plasmasonden, wie z. B. Plasmakoagulationssonden oder Plasmaablationssonden, sind allgemein bekannt. Sie sind medizinische Instrumente, die in der Elektrochirurgie verwendet werden, um biologisches Gewebe durch die Anwendung von Plasma zu koagulieren oder abzutragen. Die Koagulation dient medizinisch der Versiegelung oder Verödung von Gewebe. Im Gegensatz dazu dient die Ablation medizinisch der gezielten Abtragung oder Zerstörung von Gewebe. Insbesondere bei bestimmten endoskopischen Anwendungen ist eine möglichst großflächige Behandlung des Gewebes vorteilhaft, um die Dauer des medizinischen Eingriffs zu verkürzen.

In der WO 2019/089392 A1 wird eine Kathetervorrichtung zum Abtragen von Schleimhaut mittels Argonplasma beschrieben. Die Kathetervorrichtung weist eine oder mehrere Elektroden sowie eine aufspreizbare Korbstruktur auf, die aus Schläuchen oder einem Netz bestehen kann und durch die eine gleichmäßige Freisetzung des Behandlungsgases ermöglicht wird. In einer anderen Ausführungsform wird der Gasstrom des Behandlungsgases mittels eines doppelwandigen Ballons gleichmäßig verteilt.

In der EP 4 282 363 A2 wird eine Vorrichtung zur Gewebeablation beschrieben, die aufspreizbare, käfigförmige Elektroden aufweist. Die käfigförmigen Elektroden können verschiebbar und/oder zurückziehbar sowie radial aufspreizbar sein. Die radial ausfahrbaren Elektroden können einen oder mehrere Ballons, einen Käfig und radial ausfahrbare Arme aufweisen.

Die DE 103 50 709 A1 beschreibt zudem ein Instrument zur Koagulation von Gewebe, das zur Zufuhr von Argongas in einen Raum zwischen einer Elektrode innerhalb der Sonde und dem Gewebe ausgebildet ist. Dabei wird eine Leitvorrichtung zum Umlenken des Gasstroms in eine vorbestimmte Richtung beschrieben.

Ferner wird in der EP 3 769 707 A1 ein medizinisches Instrument beschrieben, bei dem die Elektrode mit einer Wärmeableiteinrichtung versehen ist, sodass der Wärmewiderstand der Elektrode gemessen in Längsrichtung erhöht werden kann.

Davon ausgehend ist es Aufgabe der Erfindung, eine verbesserte Plasmasonde bereitzustellen, mit der insbesondere eine größere Behandlungsfläche erreicht werden soll, die ein lageunabhängiges Zünden des Plasmas ermöglichen soll.

Die Aufgabe wird insbesondere durch die Plasmasonde gemäß Anspruch 1 gelöst.

Die erfindungsgemäße Plasmasonde ist zur Koagulation von biologischem Gewebe eines Patienten eingerichtet.

Die Plasmasonde weist einen Schlauch, der sich von einem proximalen Schlauchende zu einem distalen Schlauchende erstreckt, und einen an dem distalen Schlauchende angeordneten Sondenkopf auf.

Der Sondenkopf der Plasmasonde weist einen Elektrodenkäfig, mindestens ein Gasauslass, ein zentrales Mittelelement und ein Zentrierelement auf.

Der Elektrodenkäfig ist aus mehreren Elektroden gebildet und erstreckt sich von einem proximalen Kopfende zu einem distalen Kopfende. Vorzugsweise sind die Elektroden in einer Vorzugsposition derart angeordnet, dass zumindest ein Abschnitt des Elektrodenkäfigs seitlich über den Außenumfang des Schlauches hinausragt. Die Vorzugsposition der Elektroden des Elektrodenkäfigs ist dabei insbesondere diejenige räumliche Position und/oder Ausrichtung, in der die Elektroden ohne Einwirkung äußerer Kräfte von selbst zur Ruhe kommen. In dieser Vorzugsposition sind die inneren Spannungen und/oder Verformungen der Elektroden minimal, so dass die Elektroden in dieser bevorzugten Stellung ohne Kraftaufwand stabil verharren.

Der Elektrodenkäfig kann beispielsweise vier, fünf, sechs oder mehr Elektroden aufweisen. Die Elektroden können aus Metalldraht, z.B. aus Edelstahl, Platin, Titan, Gold, Silber, Wolfram oder aus Graphit oder Kohlenstofffasermaterial bestehen. Die Elektroden des Elektrodenkäfigs sind an dem distalen Schlauchende vorzugsweise mit einer elektrischen Zuleitung elektrisch, und vorzugsweise thermisch, verbunden. Die elektrische Zuleitung verläuft in dem Schlauch von dem distalen Schlauchende bis zum proximalen Schlauchende, an welchem der Schlauch dazu eingerichtet ist, die elektrische Zuleitung, beispielsweise über einen bestimmten Anschlussstecker, an eine Versorgungseinrichtung anzuschließen.

Der Gasauslass ist am proximalen Kopfende angeordnet und über den Schlauch am proximalen Schlauchende mit dem abzugebenden Gas versorgbar. Der Schlauch kann mindestens einen Gaskanal aufweisen, der sich vom distalen Schlauchende bis zum proximalen Schlauchende erstreckt, wobei der Schlauch zur Verbindung des Gaskanals mit der Versorgungseinrichtung, beispielsweise über einen bestimmten Anschlussstecker, ausgebildet ist. Bei dem Gas kann es sich beispielsweise um Argon, Helium, Stickstoff oder Sauerstoff handeln.

Das zentrale Mittelelement definiert eine zentrale Achse des Sondenkopfes. Das Zentrierelement ist am distalen Ende des Sondenkopfes angeordnet, wobei die Elektroden des Elektrodenkäfigs mit dem Zentrierelement verbunden und entlang der Mittelachse relativ zu dem Mittelelement verschiebbar sind.

Das Zentrierelement kann gegenüber dem Mittelelement axial (entlang der Mittelachse) verschiebbar sein. Das Mittelelement kann eine Führung für das Zentrierelement bilden. Insbesondere sind die Elektroden am proximalen Kopfende axial unverschieblich (fest) fixiert. Das Zentrierelement kann auch axial unverschiebbar sein, wenn die Elektroden gegenüber dem Zentrierelement entlang der Mittelachse verschiebbar sind.

Das Zentrierelement kann rotationssymmetrisch zur Mittelachse scheibenförmig ausgebildet sein. Vorzugsweise weist das Zentrierelement eine Durchgangsöffnung auf. Die Durchgangsöffnung kann so bemessen sein, dass sich das Zentrierelement entlang der Mittelachse hindurch erstreckt. Vorzugsweise weist das Zentrierelement an seinem Außenumfang Anschlussstellen auf, an denen die Elektroden des Elektrodenkäfigs mit dem Zentrierelement verbunden sind. Die Elektroden sind vorzugsweise reib- und/oder formschlüssig mit dem Zentrierelement verbunden. Das Zentrierelement und der Elektrodenkäfig können auch einstückig ausgebildet sein. Das Mittelelement kann eine Führung für das Zentrierelement bilden. Insbesondere sind die Elektroden am proximalen Kopfende axial unverschieblich (fest) fixiert. Das Zentrierelement kann rotationssymmetrisch zur Mittelachse scheibenförmig ausgebildet sein. Bevorzugt weist das Zentrierelement eine Durchgangsöffnung auf. Die Durchgangsöffnung kann so bemessen sein, dass sich das Zentrierelement entlang der Mittelachse durch diese hindurch erstreckt. Insbesondere kann das Zentrierelement einstückig ausgebildet sein. Vorzugsweise weist das Zentrierelement an seinem Außenumfang Anschlussstellen auf, an denen die Elektroden der Zentriereinrichtung anschließbar sind.

Das Mittelelement erstreckt sich insbesondere von dem proximalen Kopfende zu dem distalen Kopfende der Plasmasonde. Vorzugsweise ist das Mittelelement als eine Spüldüse ausgebildet, das dazu eingerichtet ist, an dem distalen Kopfende ein Spülfluid, insbesondere eine Spülflüssigkeit, auszugeben. Das Mittelelement kann zumindest im Wesentlichen zylindrisch ausgebildet sein. In dem Mittelelement kann ein Kanal ausgebildet sein, der sich axial entlang der Mittelachse erstreckt. Der Kanal kann in eine Spülöffnung münden, die an dem distalen Kopfende des Mittelelements ausgebildet ist. Am proximalen Kopfende kann der Kanal mit einer Spülfluidzuleitung fluidisch verbunden sein. Die Spülfluidzuleitung kann sich vom distalen Schlauchende bis zum proximalen Schlauchende erstrecken. Die Spülfluidzuleitung ist insbesondere mittig im Schlauch verlaufend angeordnet. Die Spülfluidzuleitung kann ein flexibler Schlauch, wie beispielsweise eine Kapillare, sein.

Die Elektroden des Elektrodenkäfigs können durch das axial bewegliche Zentrierelement am distalen Kopfende federnd nachgeben. Dadurch können die Elektroden unabhängig vom Mittelelement im Durchmesser und axiale Position elastisch verändert werden. Beim Einführen der Plasmasonde in den Körper des Patienten, z.B. durch ein Endoskop, oder beim Herausziehen der Plasmasonde können von außen Kräfte auf den Elektrodenkörper wirken. Durch die elastische Nachgiebigkeit der Elektroden, unterstützt durch die Beweglichkeit des Zentrierelementes relativ zum Mittelelement, können die Elektroden aus der Vorzugsposition bewegt werden, wodurch der Widerstand für den Anwender beim Einführen oder Herausziehen der Plasmasonde verringert werden kann. Darüber hinaus wird das Risiko verringert, dass sich die Elektroden des Elektrodenkäfigs dauerhaft derart verbiegen, dass die Applikationsfläche kleiner wird, als wenn die Elektroden in der Vorzugsposition angeordnet sind.

Insbesondere weist der Elektrodenkäfig eine um die Mittelachse rotationssymmetrische Hüllkontur auf. Die Elektroden weisen bevorzugt Stellen auf, die in der Vorzugsposition am weitesten von der Mittelachse entfernt sind. Diese Stellen sind insbesondere innerhalb des Abschnitts angeordnet, dessen Abstand von der Mittelachse den Außenumfang des Schlauches überschreitet.

Der Elektrodenkäfig kann bauchig, insbesondere birnenförmig, ausgebildet sein. Insbesondere weist der Elektrodenkäfig einen an das distale Kopfende angrenzenden ersten Teilabschnitt und einen zweiten an das proximale Kopfende angrenzenden Teilabschnitt auf.

Der erste Teilabschnitt und der zweite Teilabschnitt des Elektrodenkäfigs können zueinander symmetrisch oder asymmetrisch ausgebildet sein. Vorzugsweise weist der erste Teilabschnitt des Elektrodenkäfigs eine größere Krümmung als der zweite Teilabschnitt des Elektrodenkäfigs auf, so dass der erste Teilabschnitt bauchiger als der zweite Teilabschnitt ausgebildet sein kann. Diese geometrische Gestaltung des Elektrodenkäfigs unterstützt die Zündfähigkeit der Plasmasonde auch bei besonders spitzen Winkeln zwischen der Gewebeoberfläche des Patienten und dem Sondenkopf der Plasmasonde, insbesondere zwischen der Gewebeoberfläche des Patienten und der Mittelachse des Sondenkopfes. Der zweite Teilabschnitt ist durch die geringere Krümmung länglicher ausgebildet als der erste Teilabschnitt, wodurch das Herausziehen des Sondenkopfes aus dem Endoskop vereinfacht werden kann. Die Elektroden können radial nach innen federn.

Es wird bevorzugt, dass der Elektrodenkäfig asymmetrisch bezüglich einer sich radial zur Mittelachse erstreckenden und durch die Stelle an dem sich in der Vorzugsposition befindlichen Elektrodenkäfig mit dem größten Außenumfang verlaufende Ebene ist. Die Stelle mit dem größten Außenumfang liegt insbesondere näher am distalen Kopfende als am proximalen Kopfende. Diese Stelle kann durch Punkte auf den Elektroden definiert sein, die über den Elektrodenverlauf vom proximalen zum distalen Kopfende den größten Abstand zur Mittelachse aufweisen. Insbesondere ist der Elektrodenkäfig asymmetrisch bzw. nicht spiegelsymmetrisch zu dieser Ebene.

Vorzugsweise besteht das Zentrierelement aus einem Material, das eine Wärmeleitfähigkeit aufweist, die gleich groß oder größer als die der Elektroden des Elektrodenkäfigs ist, so dass das Zentrierelement die beim Zündvorgang entstehende Wärme aufnehmen und abführen kann. Die Plasmasonde appliziert normalerweise berührungslos, jedoch kann bei einer großflächigen Applikationszone, d. h. Koagulations- und/oder Ablationszone, wie sie bei einem großflächigen Elektrodenkäfig vorliegt, nicht ausgeschlossen werden, dass es zu einem unbeabsichtigten Kontakt zwischen den Elektroden und dem Gewebe kommt. Das Ableiten der Wärme ermöglicht es, bei Kontakt zwischen Elektrodenkäfig bzw. Zentrierelement und Gewebe eine Anhaftung von Gewebebestandteilen und koagulierten Flüssigkeiten zu vermeiden, wodurch die Sichtbarkeit und die sichere Funktion der medizinischen Instrumente erhalten werden können. Darüber hinaus kann durch die erhöhte Wärmeleitfähigkeit des Zentrierelements die beim Zündvorgang entstehende Wärme aufgenommen und abgeführt werden. Die Gefahr einer thermischen Schädigung der distalen Komponenten wie Elektroden, Zentrierelement und Mittelelement, die durch die hohe thermische Belastung während des Zündvorgangs und der Plasmaapplikation besteht, kann somit reduziert werden.

Insbesondere ist in dem Schlauch eine elektrische Zuleitung angeordnet, die am distalen Schlauchende mit den Elektroden des Elektrodenkäfigs verbunden ist. Die elektrische Zuleitung besteht vorzugsweise aus einem Material, das eine höhere Wärmeleitfähigkeit aufweist als die Elektroden des Elektrodenkäfigs. Dies ermöglicht es, die beim Zündvorgang entstehende Wärme über die elektrische Zuleitung aufzunehmen und vom Sondenkopf weg in Richtung des proximalen Schlauchendes zu leiten bzw. zu verteilen. Die elektrische Zuleitung kann beispielsweise aus Kohlenstofffasern bestehen.

Alternativ oder zusätzlich kann die elektrische Zuleitung mit einer Beschichtung versehen sein, deren Material eine höhere Wärmeleitfähigkeit aufweist als die Elektroden des Elektrodenkäfigs. Dadurch kann die Wärmeleitfähigkeit der elektrischen Zuleitung verbessert werden. Dadurch kann die elektrische Zuleitung die beim Zündvorgang entstehende Wärme aufnehmen und vom Sondenkopf in Richtung des proximalen Schlauchendes ableiten. Beispielsweise kann die elektrische Zuleitung ein Draht mit einer Silberbeschichtung oder ein Kupferdraht mit einer Polyamidbeschichtung sein.

Aus dem am proximalen Kopfende angeordneten Gasauslass kann Gas in Richtung des distalen Kopfendes austreten. Das Mittelelement kann als Gasführungseinrichtung ausgebildet sein. Vorzugsweise ist das Mittelelement dazu eingerichtet, das aus dem Gasauslass austretende Gas seitlich (quer zur Mittelachse) in Richtung der Elektroden des Elektrodenkäfigs zu leiten. Insbesondere ist das Mittelelement derart konturiert, dass das aus dem Gasauslass austretende Gas quer zur Mittelachse (seitlich nach außen) umgelenkt wird. Dies kann zu einer Verbesserung der Gasverteilung am Sondenkopf führen, wodurch insbesondere eine verbesserte seitliche Zündfähigkeit erreicht werden kann, bei der das Gas die Elektroden des Elektrodenkörpers umgibt. Quer zur Mittelachse bedeutet insbesondere orthogonal zur Mittelachse.

Vorzugsweise variiert der Radius des Mittelelements entlang der Mittelachse so, dass das Gas seitlich nach außen strömt. Der Radius des Mittelelementes kann sich vom distalen Kopfende zum proximalen Kopfende (kontinuierlich) verjüngen. Das Mittelelement kann wenigstens eine Leitlippe aufweisen. Die Leitlippe kann insbesondere in einem Austrittsbereich des Gasauslasses derart angeordnet sein, dass der aus dem Gasauslass austretende Gasstrom auf die Leitlippe trifft. Die Leitlippe kann insbesondere Turbulenzen in der Gasströmung erzeugen, wodurch eine bessere Verteilung, insbesondere in lateraler Richtung, des ausströmenden Gases erreicht werden kann. Die Leitlippe kann dazu ausgebildet sein, den Gasstrom quer zur Mittelachse seitlich nach außen zu leiten.

Das Mittelelement kann am proximalen Kopfende mit einer Spülfluidzuleitung verbunden sein. Die Spülfluidzuleitung kann am proximalen Kopfende aus dem Schlauch herausgeführt sein. Das Mittelelement und die Spülfluidzuleitung können dann außerhalb des Schlauchs im Bereich des Sondenkopfes miteinander verbunden sein.

Insbesondere sind die Elektroden am proximalen Kopfende in einem den Gasauslass bildenden Ringspalt zwischen der Spülfluidzuleitung und dem Schlauch angeordnet. Alternativ können die Elektroden am proximalen Kopfende in einem den Gasauslass bildenden Ringspalt zwischen dem Mittelelement und dem Schlauch angeordnet sein.

Vorzugsweise können die Elektroden so angeordnet sein, dass der Ringspalt durch sie unterbrochen ist, wodurch das Gas aus mehreren Ringsegmenten zwischen den Elektroden in Richtung des distalen Kopfendes austreten kann.

Die Elektroden sind vorzugsweise am proximalen Kopfende des Sondenkopfes ortsfest, d.h. in Richtung der Mittelachse unbeweglich, befestigt. Beispielsweise können die Elektroden an dem proximalen Kopfende mittels einer Klemmverbindung ortsfest befestigt sein. Beispielsweise haben die Elektroden einen größeren Durchmesser als die Spaltbreite des Ringspalts. Dieses Übermaß kann dazu führen, dass die Elektroden zwischen dem Mittelelement und dem Schlauch oder zwischen der Spülfluidzuleitung und dem Schlauch eingeklemmt werden. In dieser Form sind die Elektroden des Elektrodenkäfigs am proximalen Kopfende fest und am distalen Kopfende relativ zum Mittelelement beweglich.

Weitere Einzelheiten, vorteilhafte Ausführungsformen der Erfindung ergeben sich aus den Unteransprüchen, Zeichnungen und der Beschreibung. Es zeigen:
Figur 1 ein Beispiel der erfindungsgemäßen Plasmasonde mit einer Versorgungseinrichtung, an das die Plasmasonde angeschlossen ist, in perspektivischer Darstellung;
Figur 2 ein Beispiel des Sondenkopfes der Plasmasonde, in perspektivischer Darstellung;
Figur 3 einen Querschnitt des Schlauchs der Plasmasonde am distalen Schlauchende;
Figur 4 einen Längsschnitt des Sondenkopfes der Plasmasonde;
Figuren 5 bis 8 weitere Beispiele des Sondenkopfes der Plasmasonde, in perspektivischer Darstellung.

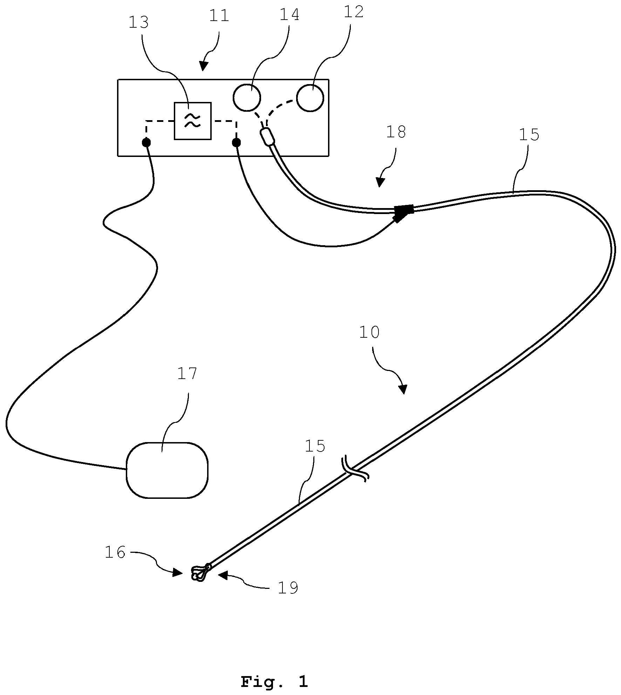

Figur 1 zeigt ein Beispiel einer erfindungsgemäßen Plasmasonde 10, die zur Koagulation von biologischem Gewebe eines Patienten eingerichtet ist, und einer Versorgungseinrichtung 11, mit der die Plasmasonde 10 verbunden ist. Die Erfindung wird in Figur 1 anhand einer Argon-Plasmasonde erläutert, die zur Koagulation von biologischem Gewebe eingerichtet ist. Das erfindungsgemäße Konzept ist jedoch auch auf jede andere Plasmasonde anwendbar.

Die Versorgungseinrichtung 11 umfasst eine Gasquelle 12, einen Generator 13 zur elektrischen Versorgung der Plasmasonde 10 und eine Spülfluidquelle 14 zur Versorgung der Plasmasonde 10 mit einem Spülfluid. Die Gasquelle 12 ist in Figur 1 eine Argongasquelle.

Die Plasmasonde 10 weist einen Schlauch 15 und einen Sondenkopf 16 auf. Der Schlauch 15 ist über entsprechende Anschlussmittel mit der Versorgungseinrichtung 11 verbunden, so dass der Sondenkopf 16 über den Schlauch 15 mit allen erforderlichen Medien versorgt wird. Die Plasmasonde 10 ist an einem proximalen Ende 18 mit der Versorgungseinrichtung 11 verbunden. Der Schlauch 15 der Plasmasonde 10 erstreckt sich von dem proximalen Schlauchende 18 bis zu einem distalen Schlauchende 19, an dem der Sondenkopf 16 der Plasmasonde 10 angeordnet ist.

Der Generator 13 erzeugt elektrische, hochfrequente (HF) Ströme, die über eine in dem Schlauch 15 verlaufende elektrische Zuleitung dem Sondenkopf 16 zugeführt werden. Der Generator 13 ist außerdem über entsprechende Verbindungsmittel mit einer Neutralelektrode 17 verbunden, die vor der Anwendung der Plasmasonde 10 am Patienten anzubringen ist. Die nachfolgende Beschreibung gilt jedoch auch für Plasmasonden mit anderen Konfigurationen der Neutralelektrode.

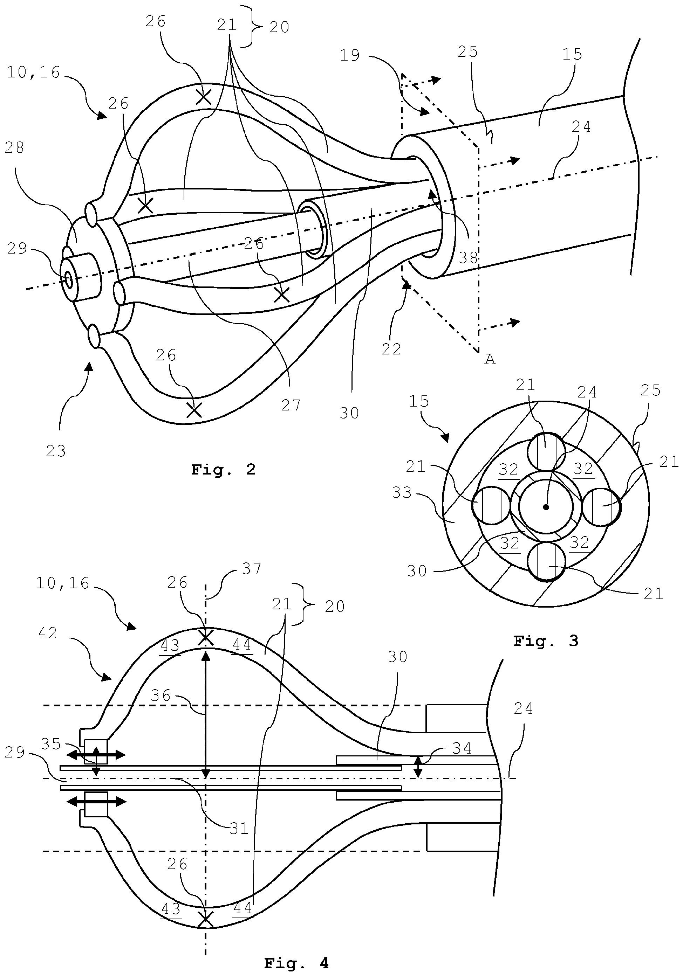

Figur 2 zeigt ein Beispiel des Sondenkopfes 16 der Plasmasonde 10 in einer perspektivischen Ansicht. Der Sondenkopf 16 weist einen Elektrodenkäfig 20 auf, der aus mehreren Elektroden 21 gebildet ist. Bei dem in Figur 2 gezeigten Beispiel besteht der Elektrodenkäfig 20 aus vier Elektroden 21. Anders als in Figur 2 gezeigt, kann der Elektrodenkäfig 20 auch aus mehr als vier Elektroden 21 ausgebildet sein. Die Elektroden 21 des Elektrodenkäfigs 20 verlaufen von einem proximalen Kopfende 22 zu einem distalen Kopfende 23 des Sondenkopfes 16.

In dem Zentrum des Elektrodenkäfigs 20 ist eine Mittelachse 24 ausgebildet, die insbesondere von dem proximalen Kopfende 22 zum distalen Kopfende 23 verläuft. Zentrisch zum Elektrodenkäfig 20 weist der Sondenkopf 16 ein Mittelelement 27 auf, das sich entlang der Mittelachse 24 von dem proximalen Kopfende 22 zu dem distalen Kopfende 23 hin erstreckt.

Die Elektroden 21 des Elektrodenkäfigs 20 ragen quer zur Mittelachse 24 über einen Außenumfang 25 des Schlauchs 15, zumindest teilweise seitlich hervor. Die einzelnen Elektroden 21 sind insbesondere konvex. Zwischen dem proximalen Kopfende 22 und dem distalen Kopfende 23 weisen sie einen Stellen 26 auf, an denen der Außenumfang der Elektroden 21 in seitliche Richtung am größten ist. An dem proximalen Kopfende 22 und dem distalen Kopfende 23 ist der Außenumfang der Elektroden 21 kleiner als an den Stellen 26.

Am distalen Kopfende 23 weist der Sondenkopf 16 zudem ein Zentrierelement 28 auf, an dem die einzelnen Elektroden 21 des Elektrodenkäfigs 20 angebracht sind. In dem in Figur 2 gezeigten Beispiel sind die Elektroden 21 an dem Zentrierelement unbeweglich angebracht. Die Elektroden 21 können an dem Zentrierelement 28 beispielsweise festgeklemmt sein. Die Verbindung kann auch stoffschlüssig sein, beispielsweise als Lötverbindung, Klebeverbindung oder sonstige vergleichbare Verbindung. Wenn die Elektroden 21 unbeweglich mit dem Zentrierelement verbunden sind, ist das Zentrierelement 28 relativ zu dem Mittelelement 27 entlang der Mittelachse 24 beweglich.

Zusätzlich oder alternativ können die Elektroden 21 mit dem Zentrierelement 28 entlang der Mittelachse 24 relativ zum Zentrierelement 28 beweglich verbunden sein. Das Zentrierelement 38 kann dann unbeweglich mit dem Mittelelement 27 verbunden sein. Das Zentrierelement 28 und das Mittelelement 27 können einstückig ausgebildet sein, wenn die Elektroden 21 relativ zum Zentrierelement 28 entlang der Mittelachse 24 bewegbar sind.

In Figur 2 ist das Zentrierelement 28 auf dem Mittelelement 27 sitzend angeordnet und relativ zu dem Mittelelement 27 verschiebbar. Die einzelnen Elektroden 21 des Elektrodenkäfigs 20 sind so an dem distalen Kopfende 23 an festen Positionen relativ zueinander angebracht. Dennoch sind die Enden der Elektroden 21 durch das Zentrierelement 28 entlang der Mittelachse 24 relativ zu dem Mittelelement 27 beweglich.

Das in Figur 2 gezeigte Mittelelement 27 ist als eine Spüldüse ausgebildet, durch welche ein Spülfluid, wie z.B. Wasser, in distaler Richtung von dem Sondenkopf 16 abgegeben werden kann.

Das Mittelelement 27 weist hierfür eine Spülöffnung 29 auf, die an dem distalen Ende 23 des Sondenkopfes 16 angeordnet ist.

In dem Mittelelement 27 kann ein Kanal 31 (wie in Figur 4 gezeigt) ausgebildet sein, der sich entlang der Mittelachse 24 erstreckt und in der Spülöffnung 29 mündet. Der Kanal 31 ist am proximalen Kopfende 22 des Sondenkopfes 16 mit einer Spülfluidzuleitung 30 fluidisch verbunden. Die Spülfluidzuleitung 30 erstreckt sich von dem distalen Schlauchende 19 bis hin zum proximalen Schlauchende 18.

Die Spülfluidzuleitung 30 kann ein Röhrchen sein, wie ein dünner, flexibler Kunststoffschlauch, z.B. ein Kapillarrohr. Die Spülfluidzuleitung 30 verläuft insbesondere zentrisch von dem distalen Schlauchende 19 im Schlauch 15 bis hin zum proximalen Schlauchende 18, wo es an eine Spülfluidquelle 14 der Versorgungseinrichtung 11 angeschlossen werden kann.

In Figur 3 ist ein Querschnitt des Schlauches 15 entlang der Schnittebene A am distalen Schlauchende 19 abgebildet.

Die Spülfluidzuleitung 30 ist zentral im Schlauch 15 angeordnet. An die Wandung der Spülfluidzuleitung 30 angrenzend sind die Elektroden 21 des Elektrodenkäfigs 20 angeordnet.

Die Elektroden 21 sind in Figur 3 gleichmäßig über den Außenumfang der Spülfluidzuleitung 30 in einem Ringspalt verteilt. Der Ringspalt kann von dem proximalen Schlauchende 18 bis hin zum distalen Schlauchende 19 verlaufen.

Zumindest am distalen Schlauchende 19 teilen die Elektroden 21 den Ringspalt in Spaltsegmente 32 auf, die den Gasauslass 38 an dem proximalen Kopfende 22 der Plasmasonde 10 bilden. Durch die Spaltsegmente 32 des Gasauslasses 38 tritt das Argongas an dem proximalen Kopfende 22 des Sondenkopfes 16 in Richtung distales Kopfende 23 aus dem Schlauch 15 aus.

Die Elektroden 21 können sich in dem Schlauch 15, wie in Figur 3 abgebildet, zumindest teilweise weiter erstrecken. In dem Schlauch 15 ist eine elektrische Zuleitung angeordnet, die am distalen Schlauchende 19 mit den Elektroden 21 des Elektrodenkäfigs 20 verbunden ist.

Die elektrische Zuleitung kann aus einem oder mehreren Einzeldrähten, Drahtgeflechten, aus Kohlenstofffasern oder durch eine metallisierte Oberfläche gebildet sein, z.B. an dem Außenumfang der Spülfluidzuleitung 30 oder dem Innenumfang des Schlauchmantels 33.

Um die einzelnen Elektroden 21 herum ist ein Schlauchmantel 33 angeordnet. Die Elektroden 21 sind insbesondere mit einem gewissen Übermaß versehen, sodass die Elektroden 21 zwischen dem Rohr 30 und dem Schlauchmantel 33 eingeklemmt sind. Die Klemmverbindung führt insbesondere dazu, dass die Enden der Elektroden 21 am proximalen Kopfende 22 in axialer Richtung entlang der Mittelachse 24 ortsfest angeordnet sind.

Figur 4 zeigt einen Längsschnitt des Sondenkopfes 16 der Plasmasonde 10. Das Zentrierelement 28 ist auf dem Mittelelement 27 in axialer Richtung verschiebbar angeordnet. Hierdurch können die Elektroden 21 des Elektrodenkäfigs 20 am distalen Kopfende 23 des Sondenkopfes 16 ausweichen, wenn Kräfte von außen auf die Elektroden einwirken. Darüber hinaus ist das als Düse ausgebildete Mittelelement 27 unabhängig von den Elektroden 21 verschiebbar.

Die Elektroden 21 verlaufen von dem proximalen Kopfende 22 zum distalen Kopfende 23, wobei ein Abschnitt 42 der Elektroden 21 seitlich zu der Mittelachse 24 über den Außenumfang 25 des Schlauchs 15 herausragt. Der Abschnitt 42, der über den Außenumfang 25 des Schlauchs 15 hinausragt, ist vorzugsweise (in Axialrichtung) länger als die übrigen Abschnitte des Elektrodenkäfigs 20.

Der Abstand 34 zwischen den Elektroden 21 und der Mittelachse 24 am proximalen Kopfende 22 ist kleiner als der Abstand 35 der Elektroden 21 zur Mittelachse 24 am distalen Kopfende 23. An der Stelle 26 ist der Abstand 36 zwischen der Elektrode 21 und der Mittelachse 24 am größten.

Der Elektrodenkäfig 20 weist einen ersten Teilabschnitt 43 und einen zweiten Teilabschnitt 44 auf. Beide Teilabschnitte 43, 44 grenzen an die Stelle 26 mit dem größten Außenumfang an. Der erste Teilabschnitt 43 grenzt an das distale Kopfende 23 und der zweite Teilabschnitt an das proximale Kopfende 22 an.

In Figur 4 weist der erste Teilabschnitt 43 eine größere Krümmung als der zweite Teilabschnitt 44 auf. Die Stelle 26 ist insbesondere nicht mittig zwischen dem proximalen Kopfende 22 und dem distalen Kopfende 23 angeordnet. Vorzugsweise liegt die Stelle 26 näher an dem distalen Kopfende 23 als an dem proximalen Kopfende 22.

Die Elektroden 21 sind nicht spiegelsymmetrisch bezüglich einer durch die Stelle 26 verlaufenden Ebene 37. Der Elektrodenkäfig 20 ist bauchig in Richtung des distalen Kopfendes 23 ausgebildet. Dies verbessert die Zündfähigkeit der Plasmasonde 10, vor allem bei vergleichsweise flachen Winkeln zwischen dem Sondenkopf 16 und dem Gewebe.

Aus dem Gasauslass 38, der in Figur 4 von den Elektroden 21 verdeckt ist, tritt Gas an dem proximalen Kopfende 22 in Richtung des distalen Kopfendes 23 aus. Das Zentrierelement 28 kann zu einer Verwirbelung des Gasstroms führen, so dass sich das Gas (proximal) vor dem Zentrierelement 28 ansammeln und seitlich quer zur Mittelachse 24 verteilen kann. Dies kann weiter vorteilhaft für das Zündverhalten der Plasmasonde 10 sein.

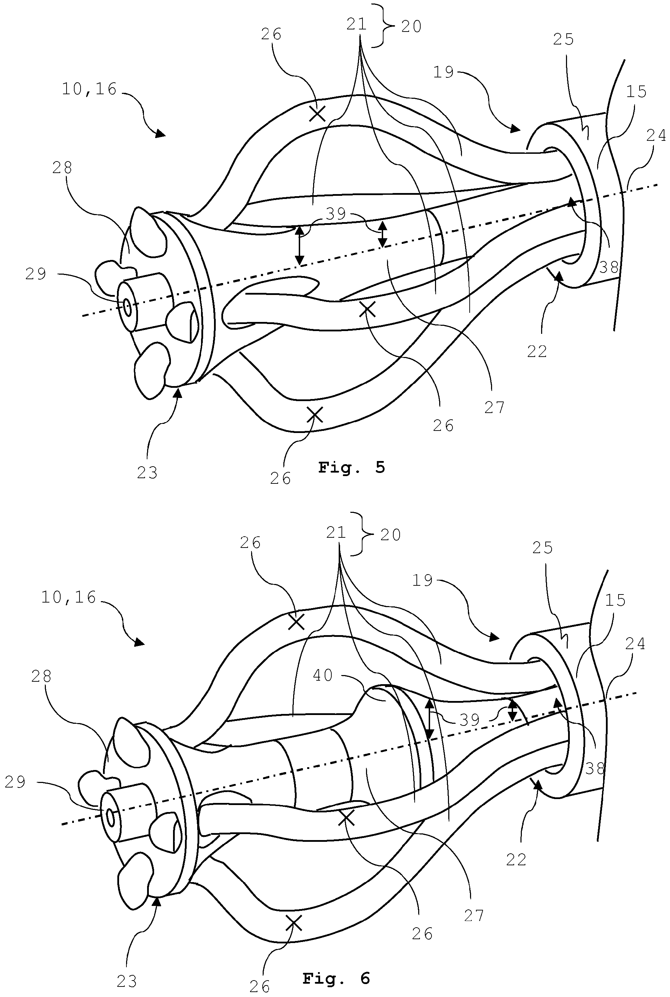

Figur 5 zeigt ein weiteres Beispiel des Sensorkopfes 16 der Plasmasonde 10. Für das in Figur 5 gezeigte Beispiel gilt das zuvor zu den Bezugszeichen Gesagte entsprechend.

Das in Figur 5 gezeigte Beispiel unterscheidet sich von dem in Figur 2 gezeigten Beispiel dadurch, dass das Mittelelement 27 dazu eingerichtet ist, den am proximalen Kopfende 22 austretenden Gasstrom quer zur Mittelachse 24 seitlich nach außen zu leiten.

Der Radius 39 des Mittelelementes 27 variiert vom proximalen Kopfende 22 zum distalen Kopfende 23 hin. In diesem Beispiel wird der Radius 39 stetig größer.

Figur 6 zeigt ein weiteres Beispiel, bei dem im Unterschied zu dem vorhergehenden Beispiel das zentrale Element 27 zusätzlich eine Leitlippe 40 aufweist, die dazu eingerichtet ist, den Gasstrom seitlich nach außen zu leiten. Die Leitlippe 40 ist zwischen der Stelle 26 und dem proximalen Kopfende 22 angeordnet, so dass der Gasstrom 38 seitlich in Richtung der Stelle 26 umgelenkt wird. Zusätzlich können durch die Leitlippe 40 Turbulenzen im austretenden Gasstrom erzeugt werden, die zu einer gleichmäßigeren Gasverteilung im Bereich des Elektrodenkäfigs 20 führen können.

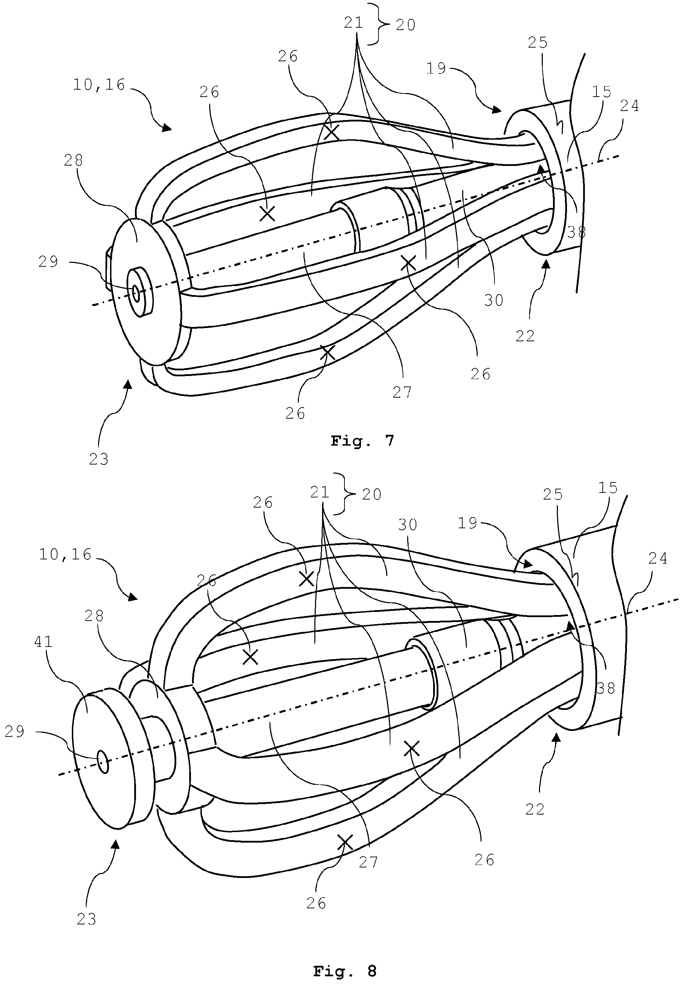

Figur 7 zeigt ein weiteres Beispiel des Sondenkopfes, bei dem die Elektroden 21 des Elektrodenkäfigs 20 sowie das Zentrierelement 28 aus einem Blechbiegeteil bestehen. Das Zentrierelement 28 und die Elektroden 21 des Elektrodenkäfigs 20 sind einteilig ausgestaltet.

Figur 8 zeigt ein weiteres Beispiel des Sondenkopfes der Plasmasonde 10. Bei dem in Figur 8 gezeigten Beispiel sind die Elektroden 21 des Elektrodenkäfigs 20 einstückig mit dem Zentrierelement 28 ausgebildet. In diesem Beispiel weist das Mittelelement 27 an dem distalen Kopfende 23 einen Anschlag 41 auf. Der Anschlag 41 ist als ein Vorsprung ausgebildet, der distal zum Zentrierelement 28 quer zur Mittelachse 24 von dem Mittelelement 27 von der Oberfläche des Mittelelements 27 vorsteht. Der Anschlag 41 begrenzt den Bewegungshub des Zentrierelements 28 ein, so dass das Zentrierelement nicht von dem als Führung dienenden Mittelelement 27 distal rutschen kann. Die Elektroden 21 und das Zentrierelement bestehen in diesem Beispiel aus Carbonfasern.

Die insofern beschriebene Plasmasonde 10 arbeitet wie folgt:

Die Plasmasonde 10 ist mit der Versorgungseinrichtung 11 verbunden, d.h. die Spülfluidquelle 14, der HF-Generator 13 und die Gasquelle 12 sind mit dem proximalen Schlauchende 18 des Schlauches 15 verbunden.

Die Plasmasonde 10 kann beispielsweise über ein Endoskop in das Körperinnere des Patienten, beispielsweise in den Magen des Patienten, eingeführt werden.

Bei derartigen Anwendungen soll insbesondere eine große Magenoberfläche behandelt werden. Bei der Ablation der Magenoberfläche wird das am proximalen Schlauchende 18 eingeleitete Gas zum distalen Schlauchende 19 transportiert, an dem der Sondenkopf 16 der Plasmasonde 10 befestigt ist. Am Sondenkopf 16 tritt der Argongasstrom aus.

Gleichzeitig werden die Elektroden 21 des Elektrodenkäfigs 20 über die Leitung 14 vom Generator 13 mit HF-Strömen versorgt. Das Argongas kann beispielsweise über das als Leitkörper ausgebildete Zwischenelement 27 seitlich zu den Elektroden 21 geleitet werden, so dass eine großflächige und winkelunabhängige Zündung und Plasmaerzeugung möglich sind.

Nach der Anwendung kann die Plasmasonde 10 über das Endoskop aus dem Magen des Patienten herausgezogen werden. Beim Einführen und Herausziehen der Plasmasonde 10 können die Elektroden 21 des Elektrodenkäfigs 20 flexibel nachgeben.

Die erfindungsgemäße Plasmasonde 10 dient zur Koagulation und/oder Ablation von biologischem Gewebe eines Patienten. Sie weist einen Schlauch 15, der sich von einem proximalen Schlauchende 18 zu einem distalen Schlauchende 19 erstreckt und einen an dem distalen Schlauchende 19 angeordneten Sondenkopf 16 auf. Der Sondenkopf 16 enthält einen Elektrodenkäfig 20, der aus mehreren Elektroden 21 gebildet ist und sich von einem proximalen Kopfende 22 zu einem distalen Kopfende 23 erstreckt. Die Elektroden 21 nehmen eine Vorzugsposition ein, in der sie zumindest in einem Abschnitt 42 seitlich über den Außenumfang 25 des Schlauches 15 hinausragen. Der Sondenkopf 16 weist außerdem ein zentrales Mittelelement 27 und ein Zentrierelement 28 auf, wobei das Mittelelement 27 eine Mittelachse 24 des Sondenkopfes 16 festlegt. An dem distalen Kopfende 23 ist das Zentrierelement 28 angeordnet. Die Elektroden 21 des Elektrodenkäfigs 20 sind entlang der Mittelachse 24 relativ zu dem Mittelelement 27 verschiebbar.

### Bezugszeichenliste

- 10: Plasmasonde
- 11: Versorgungseinrichtung
- 12: Gasquelle
- 13: Generator
- 14: Spülfluidquelle
- 15: Schlauch
- 16: Sondenkopf
- 17: Neutralelektrode
- 18: proximales Schlauchende
- 19: distales Schlauchende
- 20: Elektrodenkäfig
- 21: Elektroden des Elektrodenkäfigs
- 22: proximales Kopfende
- 23: distales Kopfende
- 24: Mittelachse
- 25: Umfang des Schlauchs
- 26: Stelle mit dem größten Außenumfang
- 27: Mittelelement
- 28: Zentrierelement
- 29: Spülöffnung
- 30: Spülfluidzuleitung (Röhrchen, Kapillarrohr)
- 31: Kanal im Mittelelement
- 32: Spaltsegment
- 33: Schlauchmantel
- 34: Abstand am proximalen Kopfende zur Mittelachse
- 35: Abstand am distalen Kopfende zur Mittelachse
- 36: Abstand an der Stelle mit dem größten Außenumfang zur Mittelachse
- 37: Ebene
- 38: Gasauslass
- 39: Radius des Mittelelements
- 40: Leitlippe
- 41: Anschlag
- 42: Abschnitt
- 43: erster Teilabschnitt
- 44: zweiter Teilabschnitt

- A: Schnittebene

## Patentansprüche

1. Plasmasonde (10), die zur Koagulation und/oder Ablation von biologischem Gewebe eines Patienten eingerichtet ist, mit einem Schlauch (15), der sich von einem proximalen Schlauchende (18) zu einem distalen Schlauchende (19) erstreckt, und einem an dem distalen Schlauchende (19) angeordneten Sondenkopf (16), aufweisend:
- einen Elektrodenkäfig (20), welcher aus mehreren Elektroden (21) gebildet ist und sich von einem proximalen Kopfende (22) zu einem distalen Kopfende (23) erstreckt,
- mindestens einen Gasauslass (38), welcher an dem proximalen Kopfende (22) angeordnet und über den Schlauch (15) an dem proximalen Schlauchende (18) mit auszugebendem Gas versorgbar ist,
- ein zentrales Mittelelement (27), welches eine Mittelachse (24) des Sondenkopfes (16) festlegt, sowie
- ein Zentrierelement (28), welches an dem distalen Kopfende (23) angeordnet ist, wobei die Elektroden (21) des Elektrodenkäfigs (20) mit dem Zentrierelement (28) verbunden und entlang der Mittelachse (24) relativ zu dem Mittelelement (27) verschiebbar sind.

2. Plasmasonde (10) nach Anspruch 1, **dadurch gekennzeichnet, dass** die Elektroden (21) des Elektrodenkäfigs (20) in einer Vorzugsposition derart angeordnet sind, dass zumindest ein Abschnitt (42) des Elektrodenkäfigs (20) seitlich über den Außenumfang (25) des Schlauches (15) hinausragt

3. Plasmasonde (10) nach Anspruch 1 oder 2, **dadurch gekennzeichnet, dass** der Elektrodenkäfig (20) einen ersten Teilabschnitt (43) und einen sich axial an den ersten Teilabschnitt (43) anschließenden zweiten Teilabschnitt (44) aufweist, wobei der erste Teilabschnitt (43) an dem distalen Kopfende (23) und der zweite Teilabschnitt (44) des Elektrodenkäfigs (20) an dem proximalen Kopfende (22) angrenzt.

4. Plasmasonde (10) nach einem der vorhergehenden Ansprüche 1 bis 3, **dadurch gekennzeichnet, dass** der Elektrodenkäfig (20) asymmetrisch bezüglich einer sich radial zur Mittelachse (24) erstreckenden und durch die Stelle an dem Elektrodenkäfig (20) mit dem größten Außenumfang verlaufende Ebene (37) ist.

5. Plasmasonde (10) nach einem der vorhergehenden Ansprüche, **dadurch gekennzeichnet, dass** das Zentrierelement (28) aus einem Material besteht, das eine Wärmeleitfähigkeit aufweist, die gleich groß oder größer als die der Elektroden (21) des Elektrodenkäfigs (20) ist.

6. Plasmasonde (10) nach einem der vorhergehenden Ansprüche, **dadurch gekennzeichnet, dass** in dem Schlauch (15) eine elektrische Zuleitung angeordnet ist, die an dem distalen Schlauchende (19) mit den Elektroden (21) des Elektrodenkäfigs (20) verbunden sind, wobei die elektrische Zuleitung aus einem Material besteht, das eine Wärmeleitfähigkeit aufweist, die gleich oder größer ist als die der Elektroden (21) des Elektrodenkäfigs (20).

7. Plasmasonde (10) nach einem der vorhergehenden Ansprüche, **dadurch gekennzeichnet, dass** die elektrische Zuleitung mit einem Überzug versehen ist, deren Material eine höhere Wärmeleitfähigkeit aufweist als das Kernmaterial der elektrischen Zuleitung und/oder das Material der Elektroden (21) des Elektrodengehäuses (20).

8. Plasmasonde (10) nach einem der vorhergehenden Ansprüche, **dadurch gekennzeichnet, dass** das Mittelelement (27) dazu eingerichtet ist, das aus dem Gasauslass austretende Gas quer zur Mittelachse (24) zu leiten.

9. Plasmasonde (10) nach einem der vorhergehenden Ansprüche, **dadurch gekennzeichnet, dass** der Außenumfang des Mittelelement (27) entlang der Mittelachse variiert, vorzugsweise vergrößert sich der Außenumfang des Mittelelements (27) von dem proximalen Kopfende (22) zum distalen Kopfende (23) hin.

10. Plasmasonde (10) nach einem der vorhergehenden Ansprüche, **dadurch gekennzeichnet, dass** das Mittelelement (27) eine Leitlippe (40) aufweist, die quer zur Mittelachse (24) von der Außenumfangsoberfläche des Mittelelements (27) vorsteht.

11. Plasmasonde (10) nach einem der vorhergehenden Ansprüche, **dadurch gekennzeichnet, dass** die Elektroden (21) an dem proximalen Kopfende (22) in einem Ringspalt angeordnet sind.

12. Plasmasonde (10) nach einem der vorhergehenden Ansprüche, **dadurch gekennzeichnet, dass** die Elektroden an dem proximalen Kopfende (22) ortsfest an dem Sondenkopf (16) befestigt sind.

13. Plasmasonde (10) nach einem der vorhergehenden Ansprüche, **dadurch gekennzeichnet, dass** das Mittelelement (27) als eine Spüldüse ausgebildet ist, die dazu eingerichtet ist, Spülfluid an dem distalen Kopfende (23) auszugeben.

14. Plasmasonde (10) nach einem der vorhergehenden Ansprüche, **dadurch gekennzeichnet, dass** eine Spülöffnung (29) an dem distalen Kopfende (23) in dem Mittelelement (27) ausgebildet ist.

15. Plasmasonde (10) nach einem der vorhergehenden Ansprüche, **dadurch gekennzeichnet, dass** ein Kanal (31) in dem Mittelelement (27) ausgebildet ist, der sich entlang der Mittelachse (24) von dem proximalen Kopfende (22) zu dem distalen Kopfende (23), insbesondere in die Spülöffnung (29) mündend, erstreckt.

16. Plasmasonde (10) nach Anspruch 15, **dadurch gekennzeichnet, dass** der Kanal (31) an dem proximalen Kopfende (22) mit einer Spülfluidzuleitung (30) fluidisch verbunden ist, die sich vorzugsweise von dem proximalen Schlauchende (18) bis zum distalen Schlauchende (19) erstreckt.
